# EUROPEAN PATENT APPLICATION

(11) **EP 3 199 036 A1**
(43) Date of publication of application: **02.08.2017**
(21) Application number: 15843997.6
(22) Date of filing: 09.07.2015
(51) Int. Cl.: A23L 33/00, C12N 1/20, A61K 8/99, A61Q 19/00

(54) **COMPOSITION, CONTAINING TYNDALLIZED LACTOBACILLUS DEAD CELLS AS ACTIVE INGREDIENT, FOR SKIN MOISTURIZING OR WRINKLE IMPROVEMENT**

(30) Priority: 23.09.2014 KR 20140126460
(71) Applicant: Korea Institute of Oriental Medicine, Daejeon 305-811 (KR)
(72) Inventor: CHAE, Sungwook, Daejeon 302-829 (KR); IM, A-Rang, Daejeon 305-811 (KR)
(74) Representative: Hoefer & Partner Patentanwälte mbB
(86) International application number: PCT/KR2015/007119
(87) International publication number: WO 2016/047907

(57) **Abstract**

The present invention relates to a composition, containing tyndallized lactobacillus dead cells as an active ingredient, for skin moisturizing or wrinkle improvement. The effect of the tyndallized lactobacillus dead cells on skin moisturizing and wrinkle improvement, which are deteriorated by ultraviolet radiation, was checked. As a result, it was confirmed that the amount of moisture evaporating from the skin was reduced and the amount of moisture contained in the skin was increased. In addition, it was confirmed that the epidermis thickness was reduced and the expression levels of wrinkle-related proteins were reduced. Therefore, the composition of the present invention improves the damage of the skin barriers, which is caused by ultraviolet radiation, thereby suppressing the loss of skin moisture or the wrinkle formation.

## Description

### [TECHNICAL FIELD]

The present invention relates to a composition for skin moisturization or wrinkle improvement, the composition comprising tyndallized dead lactic acid bacteria as an active ingredient.

### [BACKGROUND ART]

Skin is an organ that is always in contact with the outside environment. It protects the body from external physical damage and chemical substances, prevents bacteria, fungi and viruses from invading the skin, and acts as a protection barrier against the loss of skin moisture. Skin structure consists of three layers: epidermis, dermis, and subcutaneous fat. The epidermis is the thinnest of the three layers and plays an important role in moisturizing and protecting the skin. Especially, the skin is elastic and softened by moisture present in the stratum corneum of the epidermis, while it is known that the water content of 10% or more is essential for maintaining the elasticity of the stratum corneum (O.K. Jacobi, Proc.Sci.Sec.Toilet Goods Assoc. 1959, 31, 22).

Recently, exposure to strong ultraviolet rays has become more frequent due to environmental pollution and the like. Skin aging is thereby accelerated due to increased moisture loss of the skin. As the incidence of related diseases due to skin damage increases, there is a growing demand for a composition that possesses a sustained effect of maintaining a moisturizing activity and reducing wrinkles for a prolonged time in the ultraviolet-exposed skin.

On the other hand, lactic acid bacteria is a genus of bacterium that plays a beneficial role in the human body, while fermenting sugars such as carbohydrates to produce lactic acid. Up to now, around 400 types of bacteria in this genus have been found, of which 18 are known to have a commercial potential. These lactic acid bacteria have been known to enhance immune functions, especially possessing the physiological activity of enhancing the immune functions in the gastrointestinal tract. Recently, due to the so-called well-being trend, a social atmosphere, which assumes that health foods for oral consumption would also be effective via topical administration as well, has been developed. Various health functional cosmetics have been developed reportedly to possess the effect of moisturizing the skin, increasing the turnover rate of the stratum corneum of the epidermis, and reducing skin wrinkles. However, the health functional effects via the cosmetic use have been insignificant in most cases.

For example, Korean Patent Appl. Laid-Open Publication No. 1991-0019600 discloses a technology regarding a culture solution product of lactic acid bacteria for cosmetic use which is capable of moisturizing and eliminating reactive oxygen species. Korean Patent Appl. Laid-Open Publication No. 2013-0096604 discloses a fermented cosmetics composition comprising a fermented extract of germinated grains as a main ingredient and its preparation method. It discloses a technology in which a product produced by fermenting and culturing lactic acid bacteria is contained in a cosmetics composition, or in which a fermented solution obtained by fermenting germinated grains or the like with a specific lactic acid bacteria is used together with a cosmetics composition. Korean Patent Appl. Laid-Open Publication No. 2013-0115943 discloses a technology regarding antiinflammatory, skin whitening and anti-aging compositions comprising a supernatant of lactic acid bacteria lysate. However, when live lactic acid bacteria is used, there exist still problems in terms of the stability of lactic acid bacteria as well as such problems as humidification even in the form of lyophilized powder. In order to solve these problems, tyndallized dead lactic acid bacteria, which are used as the active ingredient according to the present invention, are capable of producing a product having homogeneous properties, with the advantage of a prolonged storability in a lyophilized form. The effect of tyndallized dead lactic acid bacteria on skin moisturization or wrinkle improvement has not been reported so far.

### [DETAILED DESCRIPTION OF THE INVENTION]

### [TECHNICAL PROBLEM]

The present invention has been made in view of the above-mentioned needs, and the present inventors have made intensive studies to develop a composition for skin moisturization or wrinkle improvement. As a result, the present inventors have found that tyndallized dead lactic acid bacteria suppress or reduce the loss of skin moisture, possess an excellent skin moisturizing effect of protecting a skin barrier, and inhibit the expression level of MMP-9, which is a protein involved in wrinkle formation, thus completing the present invention.

### [TECHNICAL SOLUTION]

In order to accomplish the above objects, one aspect of the present invention provides a health functional food composition for skin moisturization or wrinkle improvement, the composition comprising tyndallized dead lactic acid bacteria as an active ingredient.

In addition, another aspect of the present invention provides a cosmetic composition for skin moisturization or wrinkle improvement, the composition comprising tyndallized dead lactic acid bacteria as an active ingredient.

Furthermore, still another aspect of the present invention provides a pharmaceutical composition for skin moisturization or wrinkle improvement, the composition comprising tyndallized dead lactic acid bacteria as an active ingredient.

### [ADVANTAGEOUS EFFECT]

The present invention directs to a composition for skin moisturization or wrinkle improvement, the composition comprising tyndallized dead lactic acid bacteria as an active ingredient. The composition of the present invention is effective for skin moisturization or wrinkle improvement by suppressing or reducing the loss of skin moisture due to ultraviolet radiation. The composition of the present invention is capable of producing a product having homogeneous properties, with the advantage of a prolonged storability.

### [BRIEF DESCRIPTION OF THE DRAWINGS]

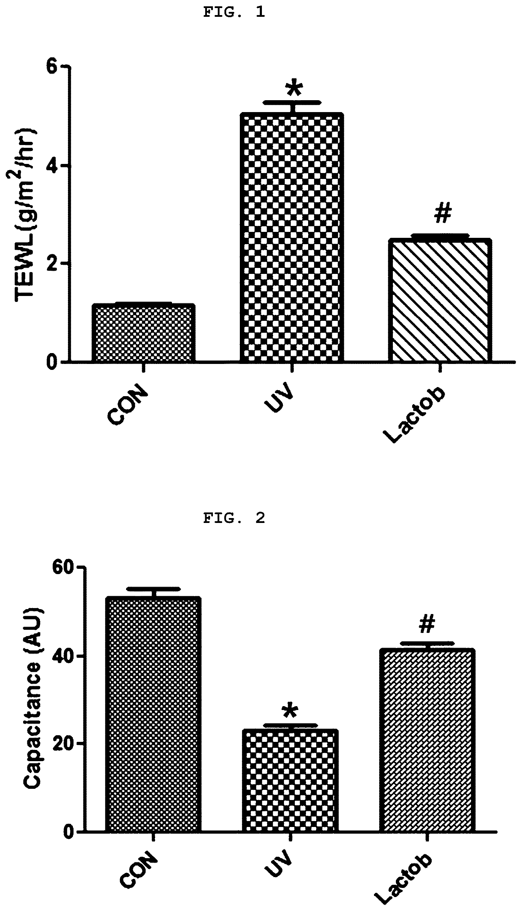

FIG. 1 shows the results of confirming the effect of tyndallized dead lactic acid bacteria on inhibiting the transdermal water loss (TDWL). * means a statistical significance compared to the control group (P <0.0001); # means a statistical significance compared to ultraviolet-irradiated group (P <0.0001). "CON" means the control group; "UV" means ultraviolet-irradiated group; "Lactob" means a group treated with tyndallized dead lactic acid bacteria.
FIG. 2 shows the results of evaluating the effect of tyndallized dead lactic acid bacteria on the skin moisture content. * means a statistical significance compared to the control group (P <0.0001); # means a statistical significance compared to ultraviolet-irradiated group (P <0.0001). "CON" means the control group; "UV" means ultraviolet-irradiated group; "Lactob" means a group treated with tyndallized dead lactic acid bacteria.

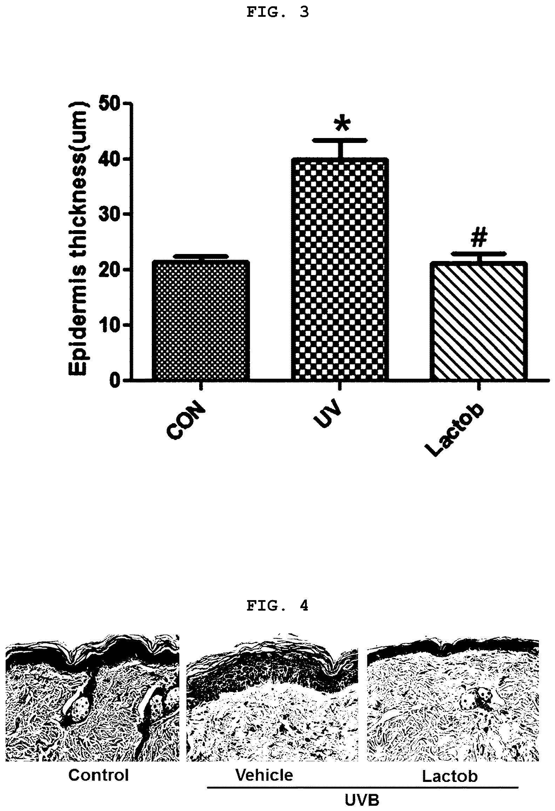

FIG. 3 shows the results of evaluating the effect of improving wrinkles by measuring changes in skin thickness. * means a statistical significance compared to the control group (P <0.0001); # means a statistical significance compared to ultraviolet-irradiated group (P <0.0001). "CON" means the control group; "UV" means ultraviolet-irradiated group; "Lactob" means a group treated with tyndallized dead lactic acid bacteria.
FIG. 4 shows the results of evaluating the effect of improving wrinkles through H&E staining. "CON" means the normal control group; "Vehicle" means the ultraviolet-irradiated group; "Lactob" means a group treated with tyndallized dead lactic acid bacteria.

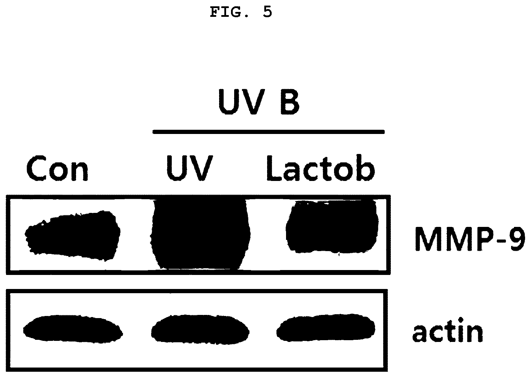

FIG. 5 shows the results of determining the expression level of a wrinkle-related protein by tyndallized dead lactic acid bacteria. "CON" means the control group; "UV" means the ultraviolet-irradiated group; "Lactob" means a group treated with tyndallized dead lactic acid bacteria.

### [BEST MODE FOR CARRYING OUT THE INVENTION]

The present invention provides a health functional food composition for skin moisturization or wrinkle improvement, the composition comprising tyndallized dead lactic acid bacteria as an active ingredient.

As used herein, the lactic acid bacteria preferably includes, but is not limited to, at least one selected from the group consisting of *Streptococcus* genus, *Lactococcus* genus, *Enterococcus* genus, *Lactobacillus* genus, *Pediococcus* genus, *Leuconostoc* genus, *Weissella* genus, and *Bifidobacterium* genus. The lactic acid bacteria is more preferably *Lactobacillus acidophilus.*

The fermentation process of lactic acid bacteria is not limited to, but preferably a process in which lactic acid bacteria is fermented after the addition of saccharide yeast extract, soy peptone and ion components, and other growth factors.

As used herein, tyndallization of lactic acid bacteria is a process for killing live lactic acid bacteria. Any known method for tyndallization may be used without limitation, while commercially available tyndallized dead lactic acid bacteria may also be used. A preferred method for tyndallizing fermented solution of lactic acid bacteria is a tyndallization process using an intermittent sterilization method, i.e., separating fermented lactic acid bacteria from fermented filtrate; and heating the fermented cells of lactic acid bacteria at 60 to 160 °C for 10 to 60 minutes, and rapidly cooling to 20 to 40 °C or below 20 °C to kill the fermented lactic acid bacteria.

As used herein, the tyndallized dead lactic acid bacteria are preferably in a lyophilized powder form, but are not limited thereto.

As used herein, the skin moisturization may include, but is not limited to, the suppression or reduction of skin moisture loss caused by ultraviolet irradiation.

As used herein, the health functional food composition may be prepared by adding the tyndallized dead lactic acid bacteria as they are, or may be prepared suitably together with other food compositions or food ingredients using typical conventional methods. Examples of the food composition in which the tyndallized dead lactic acid bacteria may be contained may include caramel, meat, sausage, bread, chocolate, candy, snacks, confectionery, pizza, ramen, other noodles, gums, dairy products including ice cream, various soups, beverages, tea, drinks, alcoholic beverages and vitamin complexes, while including all types of conventional health functional foods. That is, the kind of the above food composition is not particularly limited.

The health functional food composition may include various nutrients, vitamins, minerals (electrolytes), synthetic and natural flavors, colorants and enhancers (cheese, chocolate etc.), pectic acid and its salts, alkynic acid and its salts, organic acid, protective colloidal viscosity agents, PH adjusting agents, stabilizers, preservatives, glycerin, alcohols, carbonating agents used in carbonated drinks, and the like. It may also contain pulps for the production of natural fruit juices and vegetable drinks. The above components may be used independently or in combination.

In addition, the health functional food composition of the present invention may further contain various flavors or natural carbohydrates as an additional ingredient. The natural carbohydrates may include monosaccharides such as glucose and fructose, disaccharides such as maltose and sucrose, polysaccharides such as dextrin and cyclodextrin, and sugar alcohols such as xylitol, sorbitol and erythritol. Although the ratio of the natural carbohydrate is not critical, it is preferably 0.01 to 0.04 g, more preferably 0.02 to 0.03 g, per 100 g of the health functional food composition of the present invention, but is not limited thereto.

As the sweetening agent, natural sweetening agents such as thaumatin and stevia extract, synthetic sweetening agents such as saccharin and aspartame, and the like can be used.

The health functional food composition of the present invention may further include known skin health-related components in addition to the above-mentioned tyndallized dead lactic acid bacteria. The inclusion of a raw material which contributes to skin health will further enhance the effect of the health functional food composition according to the present invention on skin moisturization or wrinkle improvement. When the skin health-related components are further added, such factors as safety to the skin, ease of formulation, and stability of the active ingredients may be considered.

The additional skin moisturizing or wrinkle improving ingredient may be a known raw material notified as health functional food by the Korea Food & Drug Administration, or an individually recognized raw material.

The above-mentioned individually recognized raw materials preferably include, but are not limited to, complexes such as pine bark extract, red ginseng, hedge parsley, mixed extract of *Cornus officinalis,* finger root extract powder, N-acetylglucosamine, hyaluronic acid, konjac potato extract, AP collagenase-digested peptide, *Ganoderma lucidum* extract powder, and mixed extract of dandelion, and peptides.

In addition, the present invention provides a cosmetic composition for skin moisturization or wrinkle improvement, the composition comprising tyndallized dead lactic acid bacteria as an active ingredient.

As used herein, the lactic acid bacteria preferably includes, but is not limited to, at least one selected from the group consisting of *Streptococcus* genus, *Lactococcus* genus, *Enterococcus* genus, *Lactobacillus* genus, *Pediococcus* genus, *Leuconostoc* genus, *Weissella* genus, and *Bifidobacterium* genus. The lactic acid bacteria is more preferably *Lactobacillus acidophilus.*

The fermentation process of lactic acid bacteria is not limited to, but preferably a process in which lactic acid bacteria is fermented after the addition of saccharide yeast extract, soy peptone and ion components, and other growth factors.

As used herein, tyndallization of lactic acid bacteria is a process for killing live bacteria. Any known method for tyndallization may be used without limitation, while commercially available tyndallized dead lactic acid bacteria may also be used. A preferred method for tyndallizing fermented solution of lactic acid bacteria is a tyndallization process using an intermittent sterilization method, i.e., isolating fermented lactic acid bacteria from fermented solution of lactic acid bacteria; and treating the fermented lactic acid bacteria at 60 to 160 °C for 10 to 60 minutes, and rapidly cooling to 20 to 40 °C or below 20 °C to kill the fermented lactic acid bacteria.

As used herein, the tyndallized dead lactic acid bacteria are preferably in a lyophilized powder form, but are not limited thereto.

As used herein, the skin moisturization may include, but is not limited to, the suppression or reduction of skin moisture loss caused by ultraviolet radiation.

As used herein, the cosmetic composition may be prepared in the form of solution, gel, solid or dough anhydride product, emulsion produced by dispersing an oil phase in a water phase, suspension, microemulsion, microcapsule, spherical microgranule, ionic liposome, non-ionic dispersing vesicle, cream, skin toner, lotion, powder, ointment, essences, spray and concealer stick. The cosmetic composition may be prepared in the form of an aerosol composition which further contains a foam or compressed propellant.

The cosmetic composition may further comprise conventional excipients in the cosmetics field such as a fatty substance, an organic solvent, a solubilizing agent, a thickener and a gelling agent, a softener, an antioxidant, a suspending agent, a stabilizer, a foaming agent, an air freshener, a surfactant, water, an ionic or non-ionic emulsifying agent, a filler, a sequestering agent and a chelating agent, a preservative, a vitamin, a blocker, a moisturizing agent, an essential oil, a dye, a pigment, a hydrophilic or lipophilic active agent, a lipid vesicle, and other ingredients commonly used in the cosmetics field.

In the cosmetic composition for skin moisturization or wrinkle improvement comprising the tyndallized dead lactic acid bacteria according to the present invention as an active ingredient, the tyndallized lactic acid bacteria of the present invention is contained in an amount of 0.01 to 15% by weight, preferably 2 to 10% by weight.

The cosmetic composition for skin moisturization or wrinkle improvement according to the present invention may further include known skin moisturizing or wrinkle improving components in addition to the above-mentioned tyndallized dead lactic acid bacteria. The inclusion of a raw material which contributes to skin health will further enhance the effect of the cosmetic composition according to the present invention on skin moisturization or wrinkle improvement. When the skin health-related components are further added, such factors as safety to the skin, ease of formulation, and stability of the active ingredients may be considered.

Furthermore, the present invention provides a pharmaceutical composition for skin moisturization or wrinkle improvement, the composition comprising tyndallized dead lactic acid bacteria as an active ingredient.

As used herein, the lactic acid bacteria preferably includes, but is not limited to, at least one selected from the group consisting of *Streptococcus* genus, *Lactococcus* genus, *Enterococcus* genus, *Lactobacillus* genus, *Pediococcus* genus, *Leuconostoc* genus, *Weissella* genus, and *Bifidobacterium* genus. The lactic acid bacteria is more preferably *Lactobacillus acidophilus.*

The fermentation process of lactic acid bacteria is not limited to, but preferably a process in which lactic acid bacteria is fermented after the addition of saccharide yeast extract, soy peptone and ion components, and other growth factors.

As used herein, tyndallization of lactic acid bacteria is a process for killing live lactic acid bacteria. Any known method for tyndallization may be used without limitation, while commercially available tyndallized dead lactic acid bacteria may also be used. A preferred method for tyndallizing fermented solution of lactic acid bacteria is a tyndallization process using an intermittent sterilization method, i.e., isolating fermented lactic acid bacteria from fermented solution of lactic acid bacteria; and treating the fermented lactic acid bacteria at 60 to 160 °C for 10 to 60 minutes, and rapidly cooling to 20 to 40 °C or below 20 °C to kill the fermented lactic acid bacteria.

As used herein, the tyndallized dead lactic acid bacteria are preferably in a lyophilized powder form, but are not limited thereto.

The pharmaceutical composition of the present invention may further comprise at least one pharmaceutically acceptable carrier in addition to the above-described active ingredient. The pharmaceutically acceptable carrier should be compatible with the active ingredient of the present invention and may be prepared by mixing at least one of saline, sterilized water, Ringer's solution, buffered saline, dextrose solution, maltodextrin solution, glycerol, ethanol, and a mixture thereof, while other conventional additives such as antioxidants, buffers, and bacteriostatics may be also added if desired. In addition, a diluent, a dispersant, a surfactant, a binder, or a lubricant may be additionally added to formulate the composition into an injectable preparation such as an aqueous solution, a suspension and an emulsion.

In addition, it can be formulated into various forms such as powders, tablets, capsules, liquids, injections, ointments, syrups, etc. and may be provided in unit-dose or multi-dose containers such as sealed ampules and bottles.

The pharmaceutical composition of the present invention can be administered orally or parenterally. The route of administration of the pharmaceutical composition according to the present invention may include, but is not limited to, oral, intravenous, intramuscular, intraarterial, intramedullary, intradermal, intracardiac, transdermal, subcutaneous, intraperitoneal, intraenterial, intragastrointestinal, sublingual or topical route.

For such clinical administration, the pharmaceutical composition of the present invention can be formulated into a suitable formulation using known techniques. The dosage of the composition of the present invention may be easily determined by one skilled in the art, and varies depending on such factors as the patient's body weight, age, sex, health condition, diet, administration time, method, excretion rate, severity of disease, and the like.

Hereinafter, the present invention will be described in more detail with reference to Examples. It is to be understood by those skilled in the art that these Examples are only for illustrating some embodiments of the present invention more specifically and that the scope of the present invention is not limited thereto.

### Materials and methods

Tyndallized dead lactic acid bacteria were prepared by isolating fermented lactic acid bacteria of *Lactobacillus acidophilus* from its fermented solution, treating the fermented lactic acid bacteria for 30 minutes at 120 °C, and rapidly cooling to 20 °C or below to kill the fermented lactic acid bacteria.

### Experimental animals and sample administration

Hairless 7-week-old male mice (male HR-1, hairless mice, Japan SLC Inc.) were purchased from Central Lab Animal Inc. and acclimated for 1 week prior to the present experiments. During the period of acclimation, their normal state was observed and healthy animals were chosen for the experiments. The breeding environment was maintained at a temperature of 23 ± 3°C, a humidity of 50 ± 5%, and a dark-light cycle of 12 hours (07:00-19:00/lighting time). During the test period, the test animals were divided into groups of 7 animals per each group. The animals were fed freely with 5L79 (Charles river, USA) which is a special mouse feed, while UV-treated tap water was provided as a drinking water.

The animals were divided into three groups for the experiment: the control group (CON), the UV-treated group (UV), the group treated with the tyndallized dead lactic acid bacteria (Lactob). Samples were administered orally using a mouse zonde. The administration period was a total of 12 weeks, 5 days per week.

### UV radiation

Ultraviolet radiation was performed for test groups three times a week for 12 weeks, except for the control group. UVB lamp (Mineralight UV Display lamp, UVP, USA) was used for ultraviolet radiation. During a total of 12 weeks, the ultraviolet radiation dose was 60 mJ/cm² for 1^{st} to 7^{th} weeks, and 90 mJ/cm² for 8^{th} to 12^{th} weeks. Ultraviolet radiation dose was measured by using optical meter (Delta OHM, Italy), while radiation time was accordingly adjusted.

### Evaluation on the efficacy of moisturization

### 1) Evaluation of transepidermal water loss (TEWL)

The amount of transdermal water evaporation was calculated based on the area and time by using Tewameter (Courage & Khazaka, Germany) at constant temperature and humidity (23°C, 50% relative humidity), while the amount of water evaporation (G/m²/hr) was read by an electronic moisture sensor and quantified to measure the moisturizing capability of the skin.

### 2) Measurement of skin moisture

Moisture content in the skin was measured using a Corneometer (Courage & Khazaka, Germany) under constant temperature and humidity conditions (23°C, 50% relative humidity). The amount of water present in the epidermis of the skin was determined by measuring and quantifying the degree of ion of water using a sensor to calculate the amount of water and determine the moisturizing capability of the skin.

### Histological observation of skin

After removing the dorsal skin tissues of the hairless mice and fixing them in 10% neutral formalin solution, they were washed, de-watered, transpared and permeated by a conventional method, embedded in paraffin, cut into pieces of 4 µm thickness, and stained with hematoxylin & Eosin (H&E).

### Electrophoresis and western blotting

20 to 30 mg of the skin tissues were pulverized by adding a specific protein extracting-solution. A specific protein was then extracted and quantitated, and the same amount of the protein as the control group (actin) was electrophoretically transferred to the PVDF membrane and reacted with its antibody. The expression level of the protein was subsequently measured.

### [Statistical Method]

The results obtained in the Examples of the present invention were analyzed for a statistical significance among test groups and control group by using one-way ANOVA and Student t-test (p <0.05).

### Example 1: Evaluation of the moisturizing effect of tyndallized dead lactic acid bacteria

### 1) Analysis of transepidermal water loss (TEWL)

In order to confirm the moisturizing effect, transdermal water loss analysis was performed using the dorsal skin of the hairless mice in each experimental group. The amount of transdermal water loss refers to the amount of water evaporated from the skin. The higher the value, the lower the moisturizing function of the skin, indicating that the barrier function inherent to the skin is impaired. The amount of transdermal water evaporation was calculated based on the area and time by using Tewameter (Courage & Khazaka, Germany) at constant temperature and humidity (23°C, 50% relative humidity), while the amount of water evaporation (G/m²/hr) was read by an electronic moisture sensor and quantified to measure the moisturizing capability of the skin. The results are shown in FIG. 1. As shown in FIG. 1, it was confirmed that the moisturizing function of the skin was impaired due to UV-induced damage to the skin barrier (p <0.0001), and that the UV-induced skin water loss was significantly reduced by the treatment of tyndallized dead lactic acid bacteria(Lactob) (p <0.0001).

### 2) Measurement of skin moisture

In order to measure the amount of water contained in the skin, skin moisture analysis was performed on the dorsal skin of hairless mice of each experimental group. As shown in FIG. 2, the skin moisture of the UV-irradiated group was decreased compared to the control group (p < 0.0001), while the tyndallized dead lactic acid bacteria-treated group showed a higher water content than that of the UV-irradiated group. Based on these results, it was verified that the tyndallized dead lactic acid bacteria according to the present invention are effective in moisturizing the skin.

### Example 2: Evaluation of efficacy by change in skin thickness

The effect of wrinkle suppression was confirmed by measurement of skin thickness change. The thickness of the epidermis was measured from the keratin layer or the stratum corneum of the H&E stained skin tissue to its epidermal basement membrane by using a scaler-equipped microscope. As shown in FIG. 3, it was confirmed that the epidermal thickness was increased upon ultraviolet radiation, whereas it was decreased in the tyndallized dead lactic acid bacteria-treated group.

### Example 3: Evaluation of efficacy by tissue staining (H&E staining)

In order to verify the efficacy of suppressing UV-induced wrinkle, the skin tissues were removed from the hairless mice and were subjected to tissue staining. As shown in FIG. 4, it was confirmed that the keratin layer or the stratum corneum layer became flaky and the thickness of the epidermal layer was increased in the UV-irradiated group as compared with the control group. Among the UV-irradiated groups, the UV-irradiated group treated with the tyndallized dead lactic acid bacteria showed the less degree of flakiness in the keratin layer and the decreased epidermal thickness, in comparison with the UV-irradiated group without the treatment of the tyndallized dead lactic acid bacteria.

### Example 4: Evaluation of the expression level of MMP-9 (matrix metallopeptidase 9) protein

The expression level of MMP-9 (matrix metallopeptidase 9), which is an important protein in wrinkle formation, was measured. As shown in FIG. 5, it was found that the UV-irradiated group (UV) showed the increased expression level of MMP-9 compared to the control group (Con). In contrast, the test group treated with the tyndallized dead lactic acid bacteria (Lactob) decreased the level of MMP-9 protein expressed by ultraviolet radiation. From these results, it was verified that the tyndallized dead lactic acid bacteria are effective in improving wrinkles caused by ultraviolet radiation.

### Preparation Example 1: Preparation of skin moisturizing or anti-wrinkle cosmetics which comprises the tyndallized dead lactic acid bacteria as an active ingredient

### 1) Preparation of emollient skin lotion

An emollient skin lotion containing the tyndallized dead lactic acid bacteria according to the present invention as an active ingredient was prepared.

There was prepared an emollient skin lotion containing: 10.00 parts by weight of the tyndallized dead lactic acid bacteria, 1.00 parts by weight of 1,3-butylene glycol, 0.05 parts by weight of disodium EDTA, 0.10 parts by weight of allantoin, 0.05 parts by weight of dipotassium glycyrrhizate, 0.01 parts by weight of citric acid, 0.02 parts by weight of sodium citrate, 1.00 parts by weight of glycereth-26, 2.00 parts by weight of arbutin, 1.00 parts by weight of hydrogenated castor oil, 30.00 parts by weight of ethanol, a trace amount of a preservative, a coloring agent and a flavoring agent, and a remaining amount of distilled water.

### 2) Preparation of nutritional cream

A nutritional cream containing the tyndallized dead lactic acid bacteria according to the present invention as an active ingredient was prepared.

There was prepared a nutritional cream containing: 10.00 parts by weight of the tyndallized dead lactic acid bacteria, 7.00 parts by weight of 1,3-butylene glycol, 1.0 part by weight of glycerin, 0.1 part by weight of D-panthenol, 3.2 parts by weight of plant extract, 0.3 part by weight of magnesium aluminum silicate, 1.2 parts by weight of PEG-40 stearate, 2.0 parts by weight of stearic acid, 1.5 parts by weight of polysorbate 60, 2.0 parts by weight of lipophilic glyceryl stearate, 1.5 parts by weight of sorbitan sesquioleate, 3.0 parts by weight of cetearyl alcohol, 4.0 parts by weight of mineral oil, 3.8 parts by weight squalene, 2.8 parts by weight of carlylic/capric triglyceride, 1.8 parts by weight of vegetable oil, 0.4 parts by weight of dimethicone, a trace amount of dipotassium glycyrrhizate, allantoin, and sodium hyaruronate, an appropriate amount of tocopheryl acetate, triethanolamine, a preservative and a flavoring agent, and a remaining amount of distilled water.

### 3) Preparation of essence

According to the following composition ratio, the tyndallized dead lactic acid bacteria, purified water, methylparaben, hyaluronic acid extract and glycerin were weighed and mixed at 80 ± 2°C to obtain an aqueous phase. In addition, propylparaben, lecithin and macadamia nut oil were weighed and mixed at 80 ± 2°C to obtain an oil phase. Thereafter, the oil phase was added to the aqueous phase, and the two phases were mixed each other to prepare an emulsion-type mixture. A flavoring agent was added to the mixture, followed by cooling to 35 ± 2°C to obtain an essence comprising the tyndallized dead lactic acid bacteria.

The aqueous phase was comprised of 8.00 parts by weight of the tyndallized dead lactic acid bacteria, 7.58 parts by weight of purified water, 0.15 parts by weight of methylparaben, 2.50 parts by weight of hyaluronic acid extract, and 8.00 parts by weight of glycerin.

The oil phase was comprised of 0.10 parts by weight of propylparaben, 0.60 part by weight of lecithin, 10.0 parts by weight of macadamia nut oil, and 0.07 parts by weight of a flavoring agent as an additive.

### Preparation Example 2: Preparation of skin moisturizing or anti-wrinkle health functional food composition which comprises the tyndallized dead lactic acid bacteria as an active ingredient

### 1) Preparation of wheat flour food

0.5 to 5.0 parts by weight of the tyndallized dead lactic acid bacteria according to the present invention was added to wheat flour. The resulting mixture was used to prepare bread, cake, cookies, crackers and noodles.

### 2) Preparation of dairy products

5 to 10 parts by weight of the tyndallized dead lactic acid bacteria according to the present invention was added to milk, thereby preparing various dairy products such as butter and ice cream by using said milk.

### 3) Preparation of Sunsik

Using known methods, brown rice, barley, glutinous rice, and coix were dried and roasted, followed by being granulated into a powder having a particle size of 60 mesh by a grinder.

Using known methods, black beans, black sesame and wild sesame were dried and roasted, followed by being granulated into a powder having a particle size of 60 mesh by a grinder. The tyndallized dead lactic acid bacteria of the present invention were concentrated under a reduced pressure in a vacuum concentrator, followed by spray hot-air dryer. The resulting dried product was subjected to a grinder to obtain a dry powder.

The above grains, seeds, and tyndallized dead lactic acid bacteria were mixed in the following ratios: grains (30 parts by weight of brown rice, 15 parts by weight of coix, 20 parts by weight of barley), seeds (7 parts by weight of wild sesame, 8 parts by weight of black beans, 7 parts by weight of black sesame), and the tyndallized dead lactic acid bacteria (3 parts by weight), *Ganoderma lucidum* (0.5 parts by weight), and *Rehmannia glutinosa* (0.5 parts by weight).

### 4) Preparation of Chocolate

Chocolate containing the tyndallized dead lactic acid bacteria was prepared by mixing 30.0 parts by weight of cacao butter, 30.0 parts by weight of whole milk powder, 41.85 parts by weight of powdered sugar, 0.45 parts by weight of Lecithin, 0.1 parts by weight of a flavoring agent, and 0.6 parts by weight of the tyndallized dead lactic acid bacteria.

### 5) Preparation of health drinks

5 g of the tyndallized dead lactic acid bacteria and additives including liquid fructose (0.5%), oligosaccharide (2%), sugar (2%), table salt (0.5%), and water (75%) were homogeneously blended, followed by flash pasteurization. The resulting mixture was packaged in a small packaging container such as glass bottles and PET bottles to prepare health drinks.

### 6) Preparation of vegetable juice

Vegetable juice was prepared by adding 5 g of the tyndallized dead lactic acid bacteria into 1,000 mℓ of tomato or carrot juice.

### 7) Preparation of fruit juice

Fruit juice was prepared by adding 1 g of the tyndallized dead lactic acid bacteria into 1,000 mℓ of apple or grape juice.

## Claims

1. A health functional food composition for skin moisturization or wrinkle improvement, the composition comprising tyndallized dead lactic acid bacteria as an active ingredient.

2. The food composition of claim 1, wherein the lactic acid bacteria is at least one selected from the group consisting of *Streptococcus* genus, *Lactococcus* genus, *Enterococcus* genus, *Lactobacillus* genus, *Pediococcus* genus, *Leuconostoc* genus, *Weissella* genus, and *Bifidobacterium* genus.

3. The food composition of claim 1, wherein the tyndallized dead lactic acid bacteria are prepared by a process comprising the steps of:
isolating fermented lactic acid bacteria from fermented solution of lactic acid bacteria; and
treating the fermented lactic acid bacteria at 60 to 160 °C for 10 to 60 minutes, and rapidly cooling to 20 to 40 °C or below 20 °C to kill the fermented lactic acid bacteria.

4. A cosmetic composition for skin moisturization or wrinkle improvement, the composition comprising tyndallized dead lactic acid bacteria as an active ingredient.

5. The cosmetic composition of claim 4, wherein the lactic acid bacteria is at least one selected from the group consisting of *Streptococcus* genus, *Lactococcus* genus, *Enterococcus* genus, *Lactobacillus* genus, *Pediococcus* genus, *Leuconostoc* genus, *Weissella* genus, and *Bifidobacterium* genus.

6. The cosmetic composition of claim 4, wherein tyndallized dead lactic acid bacteria is contained in an amount of 0.01 to 15% by weight on the basis of the total weight of the composition.

7. The cosmetic composition of claim 4, wherein the tyndallized dead lactic acid bacteria are prepared by a process comprising the steps of:
isolating fermented lactic acid bacteria from fermented solution of lactic acid bacteria; and
treating the fermented lactic acid bacteria at 60 to 160 °C for 10 to 60 minutes, and rapidly cooling to 20 to 40 °C or below 20 °C to kill the fermented lactic acid bacteria.

8. The cosmetic composition of claim 4, wherein the tyndallized dead lactic acid bacteria are in the form of a lyophilized powder.

9. The cosmetic composition of claim 4, wherein the skin moisturization is the suppression or reduction of water loss in the skin caused by ultraviolet radiation.

10. The cosmetic composition of claim 4, wherein the composition further comprises at least one selected from the group consisting of a fatty substance, an organic solvent, a solubilizing agent, a thickener and a gelling agent, a softener, an antioxidant, a suspending agent, a stabilizer, a foaming agent, an air freshener, a surfactant, water, an ionic or non-ionic emulsifying agent, a filler, a sequestering agent and a chelating agent, a preservative, a vitamin, a blocker, a moisturizing agent, an essential oil, a dye, a pigment, a hydrophilic or lipophilic active agent, and a lipid vesicle.

11. The cosmetic composition of any one of claims 4 to 10, wherein the composition is in the form of at least one selected from the group consisting of solution, gel, solid or dough anhydride product, emulsion produced by dispersing an oil phase in a water phase, suspension, microemulsion, microcapsule, spherical microgranule, ionic liposome, non-ionic dispersing vesicle, cream, skin toner, lotion, powder, ointment, essences, spray and concealer stick.

12. A pharmaceutical composition for skin moisturization or wrinkle improvement, the composition comprising tyndallized dead lactic acid bacteria as an active ingredient.

13. The pharmaceutical composition of claim 12, wherein the lactic acid bacteria is at least one selected from the group consisting of *Streptococcus* genus, *Lactococcus* genus, *Enterococcus* genus, *Lactobacillus* genus, *Pediococcus* genus, *Leuconostoc* genus, *Weissella* genus, and *Bifidobacterium* genus.

14. The pharmaceutical composition of claim 12, wherein the tyndallized dead lactic acid bacteria are prepared by a process comprising the steps of:
isolating fermented lactic acid bacteria from fermented solution of lactic acid bacteria; and
treating the fermented lactic acid bacteria at 60 to 160 °C for 10 to 60 minutes, and rapidly cooling to 20 to 40 °C or below 20 °C to kill the fermented lactic acid bacteria.
